(19) Europäisches Patentamt
European Patent Office
Office européen des brevets

(11) EP 1 324 017 B1

(12) **EUROPÄISCHE PATENTSCHRIFT**

(45) Veröffentlichungstag und Bekanntmachung des Hinweises auf die Patenterteilung:
**27.10.2004 Patentblatt 2004/44**

(51) Int Cl.[7]: **G01N 21/33**, G01N 21/59

(21) Anmeldenummer: **02090417.3**

(22) Anmeldetag: **19.12.2002**

(54) **Verfahren zur Bestimmung realistischer UV-Schutzfaktoren oder Breitspektrumindizes**

Method to determine realistic UV protection factors or broad spectrum indexes

Procédé pour la détermination d'indices de protection UV réalistes ou d'indices large-bande

(84) Benannte Vertragsstaaten:
**AT BE BG CH CY CZ DE DK EE ES FI FR GB GR IE IT LI LU MC NL PT SE SI SK TR**

(30) Priorität: **20.12.2001 DE 10164469**

(43) Veröffentlichungstag der Anmeldung:
**02.07.2003 Patentblatt 2003/27**

(73) Patentinhaber: **Coty B.V.**
**2031 CC Haarlem (NL)**

(72) Erfinder:
- **Ferrero, Louis, Dr.**
  **06100 Nice (FR)**
- **Pissavini, Marc**
  **06100 Nice (FR)**
- **Zastrow, Leonhard, Prof. Dr.**
  **98000 Monaco (MC)**

(74) Vertreter: **Walter, Wolf-Jürgen et al**
**Anwaltskanzlei**
**Gulde Hengelhaupt Ziebig & Schneider**
**Wallstrasse 58/59**
**10179 Berlin (DE)**

(56) Entgegenhaltungen:
**US-A- 5 500 533**

- **SAYRE R M: "CORRELATION OF IN-VIVO TESTS, IN-VITRO SPF PREDICTIONS A SURVEY OF PUBLISHED STUDIES" COSMETICS & TOILETRIES, WHEATON, IL, US, Bd. 108, 1. Februar 1993 (1993-02-01), Seiten 111-114, XP000573062 ISSN: 0361-4387**
- **H. GERS-BARLAG ET AL: "In vitro testing to assess the UVA protection performance of sun care products" INTERNATIONAL JOURNAL OF COSMETIC SCIENCE, Bd. 23, Nr. 1, März 2001 (2001-03), Seiten 3-14, XP002240378**
- **A. SPRINGSTEEN ET AL: "Analytica Chimica Acta" IN VITRO MEASUREMENT OF SUN PROTECTION FACTOR OF SUNSCREENS BY DIFFUSE TRANSMITTANCE, Bd. 380, 1999, Seiten 155-164, XP002240379**
- **DIFFEY B L ET AL: "A NEW SUBSTRATE TO MEASURE SUNSCREEN PROTECTION FACTORS THROUGHOUT THE ULTRAVIOLET SPECTRUM" JOURNAL OF THE SOCIETY COSMETIC CHEMISTS, NEW-YORK, NY, US, Bd. 40, Nr. 3, 1. Mai 1989 (1989-05-01), Seiten 127-133, XP000571875**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Bestimmung realistischer UV-Schutzfaktoren oder Breitspektrumindizes für eine Sonnenschutzzubereitung.

Zahlreiche Experimente wurden bisher durchgeführt, um zu verstehen wie Sonnenschutzmittel gegen UV-Strahlung schützen können (z.B. Int. J. Cosmet. Sci., 7, 235-246 (1985)). Normalerweise sollte der letztendlich erreichte Schutz eines Sonnenschutzmittels das Ergebnis der UV-Absorption eines jeden Filters im Präparat sein. Der einfachste Weg zur Bestimmung, ist die Addition jeder einzelnen UV-Filter-Absorption, die in verdünnter Lösung gemessen wurde - Wellenlänge für Wellenlänge - und nach dem Lambert-Beer'schen Gesetz. Es ist allerdings dass die Korrelation zwischen den nach diesem Verfahren durch direkte berechneten Sonnenschutzfaktoren und den reellen Sonnenschutzfaktoren sehr ungenau ist: Es bestehen mehrere Größenordnungen Unterschied - viel zu hoch für eine Voraussage.

Mit der Einführung von in vitro Verfahren für Sonnenschutzfaktoren können in jüngster Zeit einfach realistische experimentelle Band UV-Daten für den gesamten erythermen Wellenlängebereich (290 bis 400 nm) erhalten werden. Diese neue instrumentelle Spektroskopie basiert auf der Messung von Streuemissionen durch einen Film eines Sonnenschutzmittelpräparats, dass direkte auf einen unebenen transparenten Träger aufgetragen ist, um die Haut Topographie nachzustellen, z. B. mit chirurgischen Band, wie Transpore®, hergestellt durch die Firma 3M. Im Ergebnis besteht eine gute Übereinstimmung zwischen den Sonnenschutzfaktoren, die aus diesen experimentellen in vitro Kurven erhalten wurden, und den in vivo Sonnenschutzfaktoren. Obwohl sich das Verfahren als sehr nützlich für den Hersteller einer Formulierung erwiesen hat, ist über die Zusammenhänge zwischen den Anteilen der aktiven Bestandteile (UV-Absorber) im Sonnenschutzmittel und der resultierenden UV-Absorption wenig bekannt. Der Hauptgrund hierfür liegt in der Nichtanwendbarkeit des Lambert-Beer'schen Gesetzes für die UV-Transmissionsspektroskopie von unregelmäßig dünnen Filmen.

Daher schien es bis zum heutigen Zeitpunkt unmöglich, einen Schutzfaktor für eine neue Grundmischung von UV-Filtern vorauszusagen. Der einzige Weg bestand darin, eine fertige Sonnenschutzformulierung bereitzustellen und diese durch in vitro Verfahren zu testen. Für diese Aufgabe haben die Hersteller von Formulierungen zahlreiche streng einzuhaltende Versuchsreihen zur Optimierung der UV-Filterkombinationen für die Sonnenschutzfaktoren oder den UVA-Schutz entwickelt.

Gegenstand der vorliegenden Erfindung ist ein neues Verfahren zur Bestimmung realistischer UV-Schutzfaktoren oder Breitspektrumindizes einer Sonnenschutzzubereitung entsprechend den Anteilen der aktiven UV-Absorber (UV-Filter), die in dem Sonnenschutzmittel enthalten sind.

Überraschenderweise wurde jetzt gefunden, dass eine mathematische Beziehungen zwischen der theoretischen Absorptionskurve des Sonnenschutzmittels, berechnet unter Anwendung des Lambert-Beer'schen Gesetzes für UV-Filterzusammensetzungen, und der experimentellen Absorptionskurve, die durch in vitro Spektroskopie erhältlich ist, besteht. Interessanterweise hängt diese Beziehung nur von wenigen Parametern ab, die alle mit der experimentellen in vitro Spektroskopie in Zusammenhang stehen:

- der Beschaffenheit und Rauheit des transparenten Trägers, auf dem ein dünner Film der Sonnenschutzzubereitung zur Erfassung der diffusen Transmission aufgetragen ist,
- der Menge des aufgetragen Sonnenschutzmittels und
- der Art der Basisformulierung (Vehikel), in der die UV-Filter enthalten sind.

Zur Simulation der Porosität und Struktur menschlicher Haut werden Träger mit einer rauhen Oberfläche ausgewählt. Wie bereits in der Literatur bekannt, spielen Unregelmäßigkeiten auf der Oberfläche eine Hauptrolle bei der Beeinflussung des Lichtschutzes eines topischen Sonnenschutzzubereitung. Die Menge des aufgetragen Sonnenschutzmittels muß genau bestimmt werden, um eine gute Korrelation mit in vivo Sonnenschutzfaktoren zu erhalten. Wie bei den realen Sonnenschutzfaktoren kann auch die Grundmischung, in der die UV-Filter eingebettet sind, den resultierenden Schutz mit beeinflussen.

Sobald die vorgenannte Beziehung erst einmal erstellt ist, ist die einzige Variable, die berücksichtigt werden muß, die theoretische Absorption, gleichgültig welche Wellenlänge damit verbunden ist. Auf diese Weise können unrealistische, nach dem Lambert-Beer'schen Gesetz berechnete UV-Daten in einfacher Weise in realistische UV-Daten konvertiert werden, von denen ausgehend alle Arten von Schutzfaktoren und Breitbandspektrenindizes berechnet werden können. Das nachfolgend Schema kann beim Verständnis der verschiedenen Berechnungsschritte helfen:

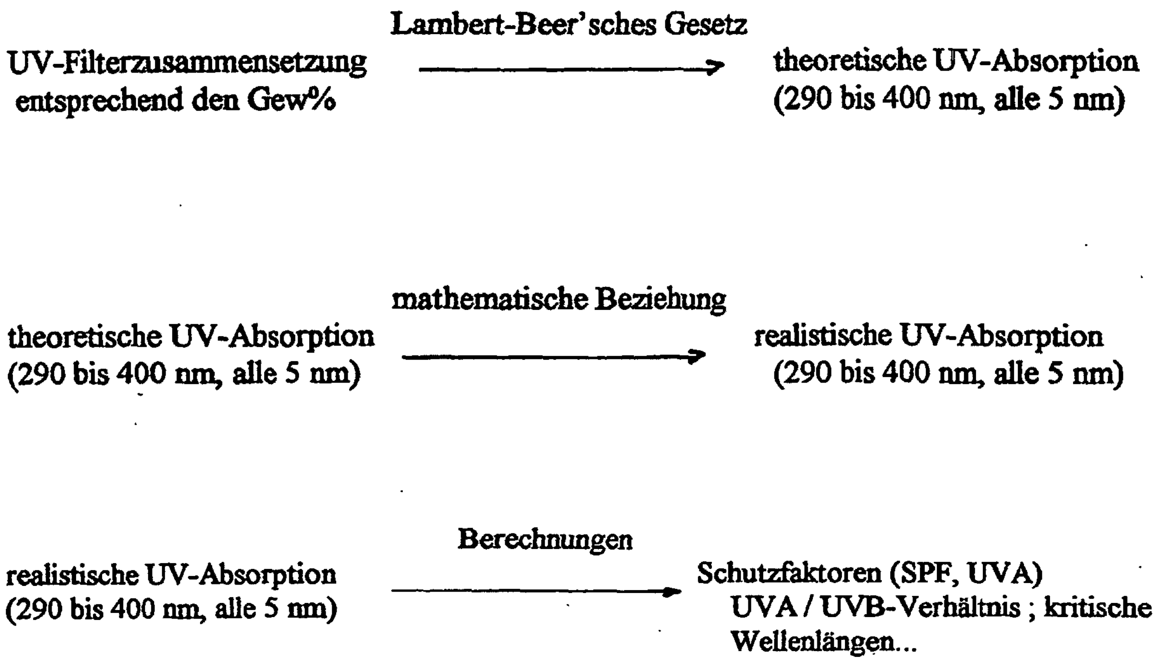


Realistische UV-Absorptionsdaten bedeuten hier, dass die berechneten Daten identisch zu denjenigen sind, die durch in vitro Messungen erhältlich sind.

Die mathematische Beziehung wird im Vorfeld bestimmt, indem experimentelle in vitro Absorptionsdaten gegen theoretische Absorptionsdaten ausgewählter Sonnenschutzmittel aufgetragen werden. Je nach verwendeten Typ der Basisformulierung - Öl/Wasser-Emulsion, Wasser/Öl-Emulsion, Gel etc. - können spezielle mathematische Beziehungen zur Berücksichtigung der Einflüsse der Basisformulierung verwendet werden.

Die Zeichnungen zeigen:

Fig. 1 UV-Absorptionsvermögen verschiedener UV-Filter bei verschiedenen Wellenlängen,

Fig.2 ein theoretisches, nach dem Lambert-Beer'schen Gesetz berechnetes UV-Spektrum,

Fig.3 die Beziehung zwischen experimentellen Absorptionsdaten und theoretischen Absorptionsdaten verschiedener Produkte F1 bis F8 und

Fig.4 ein Beispiel einer UV-Kurvensimulation entsprechend der mathematische Beziehung mit 7% Octyl Methoxycinnamate und 3% Benzophenone-3.

Das Verfahren zu Bestimmung realistischer UV-Lichtschutzfaktoren einer Sonnenschutzzubereitung gemäß der Erfindung umfasst die folgenden Schritte:

(1) Bestimmung des Absorptionsvermögens der reinen, bekannten organischen oder anorganischen UV-Filtersubstanzen in einem reinen, organischen Lösungsmittel oder einem Gemisch desselben, bei einer Wellenlänge von 290 bis 400 nm, in Wellenlängenschritten im Bereich von 1-10 nm, vorzugsweise von 5 nm;

(2) Berechnung der theoretischen UV-Absorption $A_{(\lambda)th}$ von 290 bis 400 nm, in Wellenlängenschritten im Bereich von 1-10 nm, vorzugsweise von 5 nm, einiger reiner UV-Filtersubstanzen oder deren Gemischen, die mit verschiedenen Anteilen in einer definierten Basisformulierung enthalten sind, entsprechend der Anwendung des Lambert-Beer'schen Gesetzes auf das im vorhergehenden Schritt (1) bestimmte Absorptionsvermögen unter Verwendung folgender Formel:

$$A_{(\lambda)th} = w * 1/100 * \sum_{n=1}^{n=p} K_{\lambda(n)} * a_{(n)} \qquad [1]$$

wobei n für die Anzahl der UV-Filtersubstanzen von n=1 bis n=p, w für die anfängliche Oberflächenkonzentration der Sonnenschutzzubereitung unmittelbar nach Auftragung auf einen rauhen, transparenten Träger in mg/cm$^2$, $K_{\lambda(n)}$ für das Absorptionsvermögen der Anzahl n der UV-Filtersubstanzen bei einer Wellenlänge $\lambda$ und $a_{(n)}$ für den Anteil der UV-Filtersubstanzen an der Sonnenschutzzubereitung in Gewichtsprozenten steht;

(3) Bestimmung der experimentellen UV-Absorptionen $A_{(\lambda)exp}$ von 290 bis 400 nm, in Wellenlängenschritten im Bereich von 1-10 nm, vorzugsweise von 5 nm, der UV-Filtersubstanzen oder der Gemische der Substanzen aus Schritt (2), die in demselben Anteil und in derselben Basisformulierung, wie in Schritt (2) vorliegen, wobei die Basisformulierung aus einer Gruppe vom Typ Öl/Wasser-Emulsionen, Wasser/Öl-Emulsionen, Öl, Gel, Stiftmasse, Mousse, Aerosol und Salbe ausgewählt ist und wobei die Mischung auf einem rauhen, transparenten Träger aufgetragen ist, um einen ungleichmäßigen Film mit einer Oberflächendichte von w mg/cm$^2$ zu erhalten;

(4) Auftragung eines Wertepaares der experimentellen Absorptionsdaten $A_{(\lambda)exp}$ aus Schritt (3) und der theoretischen Absorptionsdaten $A_{(\lambda)th}$ aus Schritt (2) bei derselben Wellenlänge in einen Graphen;

(5) Bestimmung einer mathematischen Funktion, die in Korrelation mit dem Graphen aus Schritt (4) steht, durch Aufnahme von Parametern über einen rauhen Film, wobei der Film ein mathematisches Modell für den Einsatz einer Sonnenschutzzubereitung auf einem transparenten Träger, wie aus Schritt (3), beschreibt und wobei durch Optimierung des Näherungsansatzes an die Wertepaare der Absorptionsdaten, $A_{(\lambda)exp}$ gegen $A_{(\lambda)th}$, mittels der Näherungsmethode der kleinsten Quadrate, verschiedene Teilareale $f_{(1)}$ bis $f_{(i)}$ mit verschiedenen Oberflächedichten $w_{(1)}$ bis $w_{(i)}$ berechnet werden, mit dem Ansatz:

$$\sum_{n=1}^{n=i} f(n) = 1$$

und

$$\sum_{n=1}^{n=i} f(n) * w(n) = w$$

wobei w für die anfängliche Oberflächendichte des Sonnenschutzmittels unmittelbar nach der Auftragung auf den transparenten Träger steht, und wobei für den Näherungsansatz gilt:

$$A_{(\lambda)exp} = -\log \left[ \sum_{n=1}^{n=i} f(n) * 10^{-w(n) * A(\lambda)th/w} \right] \qquad [2]$$

wobei $A_{(\lambda)th}$ für die berechnete theoretische Absorption nach Schritt (2) und $A_{(\lambda)exp}$ für die experimentelle Absorption nach Schritt (3) steht;

(6) Anwendung des in den Schritten (1) bis (5) beschriebenen Verfahrens zur Vorhersage realistischer Sonnenschutzfaktoren von Sonnenschutzzubereitungen, vor der experimentellen in vitro oder in vivo Bestimmung, die die folgenden Schritte umfasst:

(7) Berechnung der theoretischen UV-Absorption $A_{(\lambda)th}$ von 290 bis 400 nm, in Wellenlängenschritten im Bereich von 1-10 nm, vorzugsweise von 5 nm, einer UV-Filtersubstanz oder eines Gemisches von Substanzen, die in Gewichtsprozentanteilen $a_1$ bis $a_n$ in der Sonnenschutzzubereitung nach Schritt (6) enthalten sind, nach Formel [1] aus Schritt (2);

(8) Berechnung einer realistischen UV-Absorption $A_{(\lambda)real}$ von 290 bis 400 nm, in Wellenlängenschritten von 5 nm, entsprechend der theoretischen UV-Absorptionen aus Schritt (7) und dem Näherungsansatz [2] aus Schritt (5), in dem die Teilareale $f_{(1)}$ bis $f_{(i)}$ und die Oberflächendichten $w_{(1)}$ bis $w_{(i)}$ zuvor nach Schritt (5) für eine Basisformulierung gleichen Typs, wie bei der zu untersuchenden Sonnenschutzzubereitung, bestimmt werden

$$A_{(\lambda)real} = -\log\left[\sum_{n=1}^{n=i} f(n) * 10^{-w(n) * A(\lambda)th/w}\right] \qquad [2a]$$

(9) Berechnung von realistischen Sonnenschutzfaktoren (SPF) des Sonnenschutzmittels aus Schritt (6) nach der Formel:

$$SPF = \frac{\sum_{290}^{400} E_\lambda * I_\lambda * \Delta\lambda}{\sum_{290}^{400} E_\lambda * I_\lambda * T_\lambda * \Delta\lambda} \qquad [3]$$

wobei $T_\lambda$, erhältlich über den Zusammenhang $T_\lambda = 10^{-A(\lambda)real}$, für die Durchlässigkeit des Sonnenschutzmittels bei der Wellenlänge $\lambda$, $E_\lambda$ für die Strahlungsdichte terrestrischen Sonnenlichts der Wellenlänge $\lambda$, bei klarem Himmel, Mittags am Mittsommer und bei einer geographischen Breite von 40° N und $I_\lambda$ für das aktive Erythemspektrum steht.

Nachfolgend werden die einzelnen Schritte detailliert erläutert.

**Schritte 1 und 2: Theoretische UV-Absorptionsdaten Berechnung über das Lambert-Beer'sche Gesetz**

Wenn eine Formulierung p UV-Filter umfasst, nummeriert von n=1 bis n=p, ist die resultierenden theoretische Absorption bei einer Wellenlänge $\lambda$ und einer Oberflächendichte von w mg/cm$^2$:

$$A_{(\lambda)th} = w * 1/100 * \sum_{n=1}^{n=p} K_{\lambda(n)} * a_{(n)} \qquad [1]$$

wobei $K_{\lambda(n)}$ für das Absorptionsvermögen der Anzahl n der UV-Filtersubstanzen bei einer Wellenlänge $\lambda$ und $a_{(n)}$ für seinen prozentualen Anteil in der Sonnenschutzzubereitung steht. w korrespondiert mit der Menge an Sonnenschutzmittel (Filter) in mg/cm$^2$, die auf den in vitro Träger aufgebracht wurde. Die Berechnung wird von 290 bis 400 nm alle 5 nm wiederholt (23 Werte).

Die Absorptionsvermögen der reinen UV-Filter in verdünnter Lösung (= normalisierte Absorption bei 1 g/l und 1 cm optischer Weglänge entspricht 1 mg/cm$^2$) wurden zuvor, unter Berücksichtigung der folgenden Bedingungen, gemessen:
Arbeitsbedingungen für die Spektroskopie von reinen UV-Filtern in verdünnten Lösungen:
Ein W-VIS-Spektrometer wurde zur Datenaufzeichnung verwendet. Eine bestimmte Menge des reinen UV-Filters wurde sorgfältig in einem geeigneten Lösungsmittel gelöst (beispielsweise in einem organischen Lösungsmittel), und das Transmissionsspektrum der Lösung wurde in einer Quarzküvette gemessen. Die Absorptionsdaten (290-400 nm, 5 nm Schritte) wurden nach dem Lambert-Beer'schen Gesetz normalisiert auf 1 g/l und 1 cm optische Weglänge (Absorptionsvermögen $K_\lambda$). In den folgenden Beispielen wurde Isopropanol aufgrund seiner Polarität und UV-Durchlässigkeit verwendet, jedoch können auch andere Lösungsmittel, wie Hexan, Ethanol, n-Propanol oder veresterte kosmetische Öle, wie Caprylic/Capric Triglyceride oder Propylengylcol Dicaprylate/dicaprate (erhältlich bei der Fa. Condea Chemie unter den Namen Miglyol 812 und Miglyol 840), zum Einsatz kommen.

Eine Datenbank wird erstellt, die verschiedene Typen von geeigneten UV-Filtern umfasst, z.B.

- UVB-Filter: Octyl Methoxycinnamate; Octyocrylene; Octylsalicylate; Penylbenzylimidazole Sulfonic Acid; 4-Methylbenzylidene Camphor; Dioctylbutamido Triazone; Isoamyl p-Methoxycinnamate,
- UVA- und UVA/UVB-Filter: Benzophenone-3; Butyl Methoxydibenzoylmethane; Methylene Bis-benzoyltriazoyl Tetramethylbutylphenol und
- physikalische Filter: Zinkoxid und Titandioxid.

Die UV-Spektren einiger UV-Filter dieser Datenbank sind in der Fig. 1 dargestellt. Das Absorptionsvermögen ist gegen die Wellenlänge für jeden Absorber aufgetragen.

In der Figur 2 ist das nach Gleichung [1] berechnete theoretische UV-Spektrum eines Films eines Sonnenschutzmittel mit 2 mg/cm$^2$ aus 7 % Octyl Methoxycinnamate und 3 % Benzophenone-3 dargestellt. Die Intensität der UV-Absorption ist offensichtlich unrealistisch: die berechneten UV-Daten führen zu einem Sonnenschutzfaktoren von 41 mit einer übermäßigen Absorption von 14 bei 310 nm. Eine mathematische Funktion muss daher gefunden werden, um die unrealistischen, berechneten UV-Daten in realistische UV-Daten zu konvertieren.

**Schritte 3, 4 und 5: mathematische Annäherung**

Die Beziehung wird experimentell bestimmt, entsprechend dem transparenten Träger, der für die in vitro Spektroskopie ausgewählt wurde, der Menge an aufgetragenem Sonnenschutzmittel und dem Typ der Basisformulierung, in die der UV-Filter eingebracht ist. Die folgenden Beispiele zeigen, wie hierbei vorgegangen werden kann.

**Schritt 3: Bestimmung der experimentellen UV-Absorption**

Vorzugsweise wird als transparenter UV-Träger ein nicht fluoreszierender, photostabiler, nichtreaktiver Träger eingesetzt, der das Produkt ähnlich wie auf menschlicher Haut verteilt und der eine entsprechend texturierte Oberfläche aufweist. Ein bevorzugter Träger kann aus der Gruppe Transpore®, Vitro Skin®, aufgerauhte Quarzplatten, aufgerauhte Polymethylmethacrylat-Platten (PMMA) und ausgeschnittener menschlicher Epidermis ausgewählt werden. Im nachfolgenden Beispiel wurden Sonnenschutzmittel auf Transpore®-Band aufgetragen, um eine Oberflächendichte von 2 mg/cm$^2$ zu erzielen.

Verschiedene UV-Filter wurden getestet, entweder allein oder in Kombination, in derselben Öl/Wasser-Emulsion und entsprechend der folgenden Tabelle 1:

Tabelle 1:

| UV-Filter, die in den Formulierungen F1 bis F8 verwendet wurden: OMC = Octyl Methoxycinnamate, OS= Octylsalicylate, OC = Octyocrylene, BMDBM = Butyl Methoxydibenzoylmethane, Bz-3 = Benzophenone-3. | | | | | |
|---|---|---|---|---|---|
| UV-Filterzusammensetzung | % OMC | % OS | % OC | % BMDBM | % Bz-3 |
| F1 | **3** | 0 | 0 | 0 | 0 |
| F2 | **7** | 0 | 0 | 0 | 0 |
| F3 | 0 | **10** | 0 | 0 | 0 |
| F4 | 0 | 0 | **10** | 0 | 0 |
| F5 | 0 | 0 | 0 | **2** | 0 |
| F6 | 0 | 0 | 0 | 0 | **3** |
| F7 | **7** | 0 | 0 | 0 | **3** |
| F8 | **7** | 0 | 0 | **2** | 0 |

Experimentelle in vitro Spektroskopie:

Es wurde ein geeignetes Volumen des Sonnenschutzmittels auf den transparenten Träger aufgebrachte, um eine Oberflächendichte von 2 mg/cm$^2$ zu erzielen; siehe zur Auftragung auch J.Soc.Cosmet.Chem., 40, 127-133 (1989). Nach der Auftragung wurde das Produkt sofort über die gesamte Oberfläche verteilt unter leichtem Verstreichen

mit einem Handschuh. Die Transmissionen jedes Trägers (bei einer Wellenlänge 290 bis 400 nm und in Schritten von 5 nm) wurden mittels eines Labsphere UV-1000 S Transmissionsanalysators sorgfältig gemessen. Es wurden drei bis fünf verschiedene Träger pro Experiment eingesetzt. Ein nicht behandelter Träger wurde als Referenz verwendet.

Die experimentelle Absorption wurde direkt von den experimentellen Transmissionsdaten abgeleitet. Gleichzeitig wurde entsprechend der Formel [1] die theoretische Absorption berechnet.

**Schritte 4 und 5: mathematische Annäherung**

Wertepaare der experimentellen und theoretischen Absorption bei gleicher Wellenlänge wurden für jedes Produkt bestimmt und aufgezeichnet. Die UV-Daten der Produkte F1 bis F8 sind in der gleichen grafischen Darstellung, siehe Figur 3, zusammengefasst worden.

Erwähnenswert ist, dass die Daten auf einer einzigen Kurve liegen, gleichgültig welches Sonnenschutzmittel oder welche Wellenlänge betroffen ist. Es konnte daher ein einfacher Zusammenhang zwischen der theoretischen und experimentellen Absorption nachgewiesen werden. Bemerkenswert ist auch die fehlende Linerarität der Beziehung. Die experimentellen Daten sind stärker bei höheren Werten gedämpft als bei niedrigeren Werten. Das erklärt die spezielle Form der UV-Kurven, die über in vitro Spektroskopie erhältlich sind.

Jede mathematische Funktion, die eine gute Korrelation zwischen den Wertepaaren der experimentellen und theoretischen Absorption findet, kann verwendet werden. Unter den vielen verwendbaren Funktion (z.B. Polynome fünfter Ordnung oder Kraftfeldfunktionen) wurde bei der vorliegenden Erfindung eine spezielle Funktion gefunden, die besonders gut für diese Zwecke geeignet ist: Das ‚unebene Filmmodell', eine mathematische Näherung, die zuvor schon durch O' Neill (*J. Pharm. Sci.*, 7, 888-891 (1983)) beschrieben wurde, um den Prozentsatz des durch den unebenen Film aus absorbierenden Material transmitierten Lichts zu berechnen. Dieselbe Funktion wurde bereits zur Erklärung der speziellen Form der in vitro UV-Kurven herangezogen (Ferrero L., Orcet A.M. Zastrow L., Proceedings of the 20$^{th}$ IFSCC Congress, Cannes, France, Poster P028 (1998)). Ein einfacher Film mit nur 2 Ebenen wurde zuvor von O'Neill ist beschrieben. Ein allgemeinere Formel liegt nun vor, die nicht die Anzahl der Teilareale f(n) mit verschiedenen Oberflächendichten w(n) beschränkt. Dabei gilt:

$$\sum_{n=1}^{n=i} f(n) = 1$$

und

$$\sum_{n=1}^{n=i} f(n) * w(n) = \mathrm{w}$$

wobei w für die anfängliche Oberflächendichte des Sonnenschutzmittels unmittelbar nach der Auftragung auf den transparenten Träger steht, und wobei für den Näherungsansatz gilt:

$$A_{(\lambda)exp} = -\log\left[\sum_{n=1}^{n=i} f(n) * 10^{-w(n) * A(\lambda)th/w}\right] \qquad [2]$$

wobei $A_{(\lambda)th}$ für die berechnete theoretische Absorption nach Schritt (2) und $A_{(\lambda)exp}$ für die experimentelle Absorption nach Schritt (3) steht.

Ein unregelmäßiges Filmmodel für 3 Ebenen (n=3) führt generell zu einer verbesserten Anpassung an die UV-Daten des Sonnenschutzmittels. Die drei verschiedenen Ebenen und die drei verschiedenen Oberflächendichten werden gemäß der besten Korrelationsmethode durch die Näherungsmethode der kleinsten Quadrate bestimmt. Für die Produkte F1 bis F8, die auf dem Transpore® Band aufgetragen sind, ergibt sich beispielsweise:

$f_1$ = 0.2649 $w_1$ = 5.2216 mg/cm$^2$ mit w = 2mg/cm$^2$
$f_2$= 0.5348 $w_2$ = 1.1019 mg /cm$^2$
$f_3$ = 0.2004 $w_3$ = 0.1388 mg /cm$^2$

**Schritt 6 bis 9: Anwendung zur Voraussage von Schutzfaktoren und Breitspektrumindizes**

Die Werte für f1, f2 und f3 usw. hängen von der Beschaffenheit der Basisformulierung (z.B. Emulsion oder Gel) und den Trägereigenschaften ab. Bei letzterem spielt die Oberflächenrauhigkeit (z.B. Transpore® Ra 11,3 µm, PMMA Ra 5,7 µm, Quarz Ra 0,6 µm, Vitroskin®-Hydrat Ra 3,0 µm; Ra = arithmetischer Mittelwert der Oberflächenrauhigkeit) eine wesentliche Rolle, wobei die Oberflächenrauhigkeit durch die Menge an aufgebrachter Sonnenschutzzubereitung ausgeglichen werden kann. Die Menge an aufgebrachter Sonnenschutzzubereitung sollte im Bereich von 0,5 bis 3 $mg/cm^2$, insbesondere 1,2 bis 2 $mg/cm^2$ liegen, da so eine gute Übereinstimmung mit den in vivo Ergebnissen erzielt werden kann. In der vorliegenden Erfindung wird mit 2 $mg/cm^2$ gearbeitet.

Sobald die mathematische Beziehungen einmal ermittelt wurde, kann jede neue UV-Filterkombination auf Basis des desselben Typs des Grundgemisches ermittelt werden: Die theoretische Absorption $A_{(\lambda)th}$, berechnet nach Gleichung [1], wird in eine realistische Absorption $A_{(\lambda)real}$, über Gleichung [2a], in der die Teilareale f(n) und Oberflächendichten w(n) zuvor bestimmt wurden, überführt:

$$A_{(\lambda)real} = -\log\left[\sum_{n=1}^{n=i} f(n) * 10^{-w(n) * A(\lambda)th/w}\right] \qquad [2a]$$

Die Schutzfaktoren und Breitspektrumindizes werden dann aus der berechneten realistischen Absorption hergeleitet. Für jede Wellenlänge wird zunächst die realistische Absorption in eine Transmission umgerechnet, gemäß der einfachen mathematischen Beziehung:

$$T_{\lambda} = 10^{-A(\lambda)}$$

Alle zuvor in der Literatur beschriebenen Gleichungen für die in vitro Spektroskopie können verwendet werden (z.B. Springsteen A., Yurek R., Frazier M., Carr K.F., Anal. Chim. acta, 380, 155-164 (1999)). Beispielsweise gilt für die Berechnung des Sonnenschutzfaktors (SPF):

$$SPF = \frac{\sum_{290}^{400} E_{\lambda} * I_{\lambda} * \Delta\lambda}{\sum_{290}^{400} E_{\lambda} * I_{\lambda} * T_{\lambda} * \Delta\lambda} \qquad [3]$$

Die Wellenlängen, die durch ein Molekül oder eine Kombination von Molekülen absorbiert werden und eine spezifische photobiologische Reaktion induzieren, bilden das Anregungsspektrum für diese spezifische Reaktion.

Der UVA-Schutzfaktor bestimmt sich nach:

$$PF\ UVA = \frac{\sum_{320}^{400} E_{\lambda} * I_{\lambda} * \Delta\lambda}{\sum_{320}^{400} E_{\lambda} * I_{\lambda} * T_{\lambda} * \Delta\lambda} \qquad [4]$$

Mit denselben Variabeln wie vorhergehend, außer für $I_{\lambda}$, das auch das Anregungsspektrum für das unmittelbare Pigmentdunkeln oder das Anregungsspektrum für das anhaltende Pigmentdunkeln sein kann (biologische UVA-Endpunkte).

Das UVA/UVB-Verhältnis bestimmt sich nach der Gleichung:

$$\mathrm{UVA/UVB} = \frac{\sum_{320}^{400} A_{\lambda\,real} * \Delta\lambda}{\sum_{290}^{320} A_{\lambda\,real} * \Delta\lambda} \qquad [5]$$

Für die Berechnung der kritischen Wellenlänge λc gilt:

$$0.90 = \frac{\sum_{290}^{\lambda c} A_{\lambda} . \Delta\lambda}{\sum_{290}^{400} A_{\lambda} . \Delta\lambda} \qquad [6]$$

Die Aufzählung ist nicht abschließend. Wichtig ist die Berechnung realistischer Absorptionen, die nicht ohne die zuvor aufgestellten mathematischen Beziehungen erreichbar sind.

Der Hauptvorteil des Verfahrens ist die drastische Verringerung der Anzahl der Experimente, die bei der Entwicklung einer Sonnenschutzzubereitung benötigt werden. Der Hersteller der Formulierung kann sofort mit einem optimierten Gemisch der UV-Filter, entsprechend dem erwarteten UV-Schutz, starten. Übermäßig hohe Konzentrationen können vermieden werden, da das Verfahren es erlaubt, aus den verfügbaren UV-Filtern die beste Kombination zu wählen und deren Konzentration sorgfältig aneinander anpasst.

In einer weiteren Ansfiihrungsform der Erfindung kann bei den Schritten 4-5 das diskontinuierliche Fdmmodell durch ein kontinuierliches Filmmodell ersetzt werden mit einer unbestimmten Zahl von Oberflächendichten und Teilarealen.. Dazu wird eine Oberflächendichtefunktion $W_{(F)}$ definiert, deren Funktionsvarible F ist, der kumulative Anteil der Flächeneinheit, der eine Zahl zwischen 0 und 1 darstellt. Dabei gilt

$$\int_0^1 dF = 1 \quad ,$$

wobei dF die Teilareale f(n) ersetzt, die in dem diskontinuierlichen Modell verwendet wurden.

$$\int_0^1 W_{(F)} \times dF = \mathbf{w} \quad ,$$

wobei w die anfängliche Oberflächendichte des Sonnenschutzmittels ist nach Auftragung auf den transparenten Träger, $W_{(F)}$ ist die Oberflächendichtefunktion, and wobei der Näherungsansatz gilt:

$$A_{(\lambda)exp} = -\log\left[\int_0^1 10^{-W_{(F)} \times A\lambda th / w} \times dF\right] \qquad [2b]$$

Darin ist $A_{\lambda th}$ die berechnete theoretische Absorption nach Schritt (2), und $A_{(\lambda)exp}$ steht für die experimentelle Absorption nach Schritt (3). Eine Korrelation zwischen beiden Absorptionsdaten kann durch numerische Integration von Gleichung [2b] erreicht werden von F=0 bis F=1.

Die Oberflächendichtefunktion $W_{(F)}$ sollte unter den Funktionen bestimmt werden, um die Parameter zu bestimmen. Sonit können die Funktionsparameter durch die Näherungsmethode der kleinsten Quadrate optimiert werden, um realistische Absorptionsdaten in größerer Nähe zu den experimentellen Absorptionsdaten zu berechnen. Eine einfache polynomiale Funktion kann vorteilhaft eingesetzt werden:

$$W_{(F)} = a \times \left(p \times F^{0.5} + q \times F + r \times F^2\right)$$

mit:

$$p+q+r=1$$

und:

$$a = \frac{6 \times w}{4 \times p+3 \times q+2 \times r}.$$

Die Erfindung soll nachstehend durch Beispiele näher erläutert werden.

**Beispiele**

Um eine Voraussage für die Bestimmung realistischer Sonnenschutzfaktoren zu erreichen, muss der prozentuale Gehalt eines jeden UV-Filters und die mathematische Funktion, die die Beziehung beschreiben soll, ausgewählt werden Parameter für Gleichung [2a] im Beispiel). SPF, UVA/UVB-Verhältnisse und Schutzfaktoren UVA und UVB werden anschließend nach den Schritten 7, 8 und 9 des Verfahrens ermittelt.

**Beispiel 1**

Es wurde mit einem unregelmäßigen Filmmodell für 3 Ebenen (n=3) gearbeitet. Aus den Formulierungen F1 - F8 wurde die Formulierung F7 //% OMC, 3% Benzophenone-3) ausgewählt. Die drei verschiedenen Ebenen und die drei verschiedenen Oberflächendichten werden gemäß der besten Korrelationsmethode durch die Näherungsmethode der kleinsten Quadrate bestimmt. Es ließen sich die folgenden Werte gemäß Tabelle II und III berechnen.

| | UV FILTERS | % |
|---|---|---|
| 1 | Octyl Methoxycinnamate | 7 |
| 2 | Octocrylene | 0 |
| 3 | Octyl Salicylate | 0 |
| 4 | P B S A | 0 |
| 5 | Benzophenone-3 | 3 |
| 6 | BMDBM | 0 |
| 7 | Methylbenzilidene Camphor | 0 |
| 8 | ZnO | 0 |
| 9 | Dibenzotriazole | 0 |
| 10 | $TiO_2$ | 0 |
| 11 | Dioctyl butamido Triazone | 0 |
| 12 | Isoamyl p-methoxycinnamate | 0 |

| | |
|---|---|
| Surface density mg/cm$^2$ | 2 |
| MATHEMATICAL RELATIONSHIP | |
| $f_1$ value | 0,2649 |
| $w_1$ value | 5,2216 |
| $f_2$ value | 0,5348 |
| $w_2$ value | 1,1019 |
| $f_3$ value | 0,2004 |
| $w_3$ value | 0,1388 |
| CALCULATED SPF | 17,65 |
| RATIO UVA / UVB | 0,3049 |
| PF UVA | 2,154 |
| PF UVB | 44,733 |

Anmerkungen:

Surface density = Oberflächendichte; mathematical relationship = mathematische Beziehung; Value = Wert; calculated = berechnet; ratio = Verhältnis.

In Figur 4 sind beispielhaft die berechneten UV-Spektren und die korrespondierenden experimentellen UV-Spektren eines Gemisches aus 7%iger OMC und 3%iger 3-Benzophenon gemeinsam aufgetragen worden, um die gute Übereinstimmung der Absorptionsdaten zu demonstrieren.

**Beispiel 2**

Anwendung des Verfahrens mit den Gemischen F1 bis F8

SPF und UVA/UVB-Verhältnisse wurden für die verschiedenen UV-Filterzusammensetzungen durch Simulation erhalten. Wie in Tabelle IV ersichtlich, liegen die berechneten und die experimentell bestimmten Werte nahe beieinander.

Tabelle IV:

| Vergleich zwischen experimentellen und berechneten Werten. Experimentelle SPF und UVA / UVB-Verhältnisse wurden nach Auftragung der Substanzen aus Tabelle I auf Transpore® (2 mg/$cm^2$) gemessen. | | | | |
|---|---|---|---|---|
| UV-Filterzusammensetzung | SPF | | UVA/UVB-Verhältnis | |
| | exp. | ber. | exp. | ber. |
| F1 OMC: 3% | 6.15 | 6.46 | 0.161 | 0.195 |
| F2 OMC: 7% | 10.54 | 10.99 | 0.183 | 0.202 |
| F3 OS: 10 % | 6.02 | 4.82 | 0.148 | 0.112 |
| F4 OCT: 10% | 8.92 | 9.45 | 0.391 | 0.351 |
| F5 BMDBM: 2% | 3.41 | 3.55 | 1.479 | 1.479 |
| F6 Bz-3: 3% | 5.39 | 5.38 | 0.480 | 0.468 |
| F7 OMC: 7%<br>Bz-3: 3% | 18.39 | 17.64 | 0.328 | 0.305 |
| F8 OMC: 7%<br>BMDBM: 2 % | 25.68 | 25.78 | 0.577 | 0.574 |

**Beispiel 3**: Bestimmung der SPF und UVA/UVB-Verhältnisse, mit weiteren Produkten

Die Tabelle V enthält die UV-Daten und UV-Filterzusammensetzungen.

Tabelle V:

| Vergleich zwischen experimentellen und berechneten Werten. Experimentelle SPF und UVA / UVB-Verhältnisse wurden nach Auftragung (Sprühen) auf Transpore® (2 mg/$cm^2$) gemessen. | | | | |
|---|---|---|---|---|
| UV-Filterzusammensetzung | SPF | | UVA / UVB-Verhältnis | |
| Beispiel (2)-(8) | exp. | ber. | exp. | ber. |
| OMC : 5%<br>BZ-3: 1% (2)<br>BMDBM: 0.5% | 16.7 | 14.5 | 0.39 | 0.44 |
| OMC: 7.5%<br>Bz-3: 2% (3)<br>BMDBM: 0.5% | 25.2 | 23.2 | 0.37 | 0.40 |
| OMC: 7.5 %<br>Bz-3: 3 % (4)<br>OS: 3 %<br>BMDBM: 0.5 % | 29.6 | 27.2 | 0.39 | 0.39 |
| OMC: 7.5 %<br>Bz-3: 3 % (5)<br>OS: 3 % | 16 | 19.5 | 0.3 | 0.29 |
| OMC: 7.5%<br>Bz-3: 3 % (6)<br>OS: 4 % BMDBM: 1.4 % | 39.8 | 36.6 | 0.49 | 0.48 |

Tabelle V: (fortgesetzt)

| Vergleich zwischen experimentellen und berechneten Werten. Experimentelle SPF und UVA / UVB-Verhältnisse wurden nach Auftragung (Sprühen) auf Transpore® (2 mg/cm$^2$) gemessen. | | | | |
|---|---|---|---|---|
| UV-Filterzusammensetzung | SPF | | UVA / UVB-Verhältnis | |
| Beispiel (2)-(8) | exp. | ber. | exp. | ber. |
| OMC: 7.5 %<br>Bz-3: 5 % (7)<br>OS: 5 % BMDBM: 0.5 % | 31.3 | 33.3 | 0.4 | 0.39 |
| OMC: 7.5 %<br>Bz-3:2% (8)<br>TiO2: 5 % | 31 | 32.3 | 0.35 | 0.36 |

**Beispiel 4:**

Die Anwendung des Verfahrens mit den Gemischen F1 bis F8 erfolgte in gleicher Weise wie im Beispiel 2, jedoch mit der oben beschriebenen weiteren Ausführungsform zu Schritt 4-5 mit der Variablen F, der Funktion $W_F$ und der mathematischen Beziehung [2a].

Zum Beispiel wurden die Parameter p, q und r durch die Näherungsmethode der kleinsten Quadrate bestimmt, wodurch man eine gute Übereinstimmung mit den UV-Filterzusammensetzungen der Formulierungen F1 bis F8 erhielt:

(1) p = 0.07537
(2) q = 0
(3) r =0.92463

SPF und UVA/UVB-Verhältnisse wurden für die verschiedenen UV-F1lterzusammensetzungen durch Simulation erhalten entsprechend der die Parameter bestimmenden polynominalen Funktion. Die theoretischen Absorptionsdaten, berechnet hach Gleichung [1], werden in realistische Absorptionsdaten gemäß Gleichung [2a] umgewandelt, wie aus der folgenden Tabelle hervorgeht.

Table IVa:

| Vergleich zwischen experimentellen und berechneten Daten: SPF und UVA /UVB Verhältnis, gemessen an Produkten gemäß Tabelle I. | | | | |
|---|---|---|---|---|
| UV FilterZusammensetzung | SPF | | UVA/UVB-Verhältnis | |
| | experimentell | berechnet. | experimentell | berechnet |
| F1 OMC: 3% | 6.15 | 6.21 | 0.161 | 0.145 |
| F2 OMC: 7% | 10.54 | 9.77 | 0.183 | 0.163 |
| F3 OS: 10 % | 6.02 | 5.01 | 0.148 | 0.115 |
| F4 OCT: 10 % | 8.92 | 10.28 | 0.391 | 0.352 |
| F5 BMDBM: 2 % | 3.41 | 3.89 | 1.479 | 1.423 |
| F6 Bz-3: 3% | 5.39 | 5.61 | 0.480 | 0.478 |
| F7 OMC: 7%<br>Bz-3: 3 % | 18.39 | 17.07 | 0.328 | 0.329 |
| F8 OMC: 7%<br>BMDBM: 2% | 25.68 | 25.13 | 0.577 | 0.605 |

**Patentansprüche**

1. Verfahren zur Bestimmung realistischer UV-Lichtschutzfaktoren oder Breitbandspektrenindizes einer Sonnenschutzzubereitung, **gekennzeichnet durch** folgende Schritte:

(1) Bestimmung des Absorptionsvermögens von reinen, bekannten UV-Filtersubstanzen in einem reinen Lösungsmittel oder einem Gemisch desselben, bei einer Wellenlänge von 290 bis 400 nm, in Wellenlängenschritten im Bereich von 1-10 nm;

(2) Berechnung der theoretischen UV-Absorption $A_{(\lambda)th}$ von 290 bis 400 nm, in Wellenlängenschritten von 5 nm, einiger reiner UV-Filtersubstanzen oder von deren Gemischen, die mit verschiedenen Anteilen in einer definierten Basisformulierung enthalten sind, entsprechend der Anwendung des Lambert-Beer-Gesetzes auf das im vorhergehenden Schritt (1) bestimmte Absorptionsvermögen unter Verwendung folgender Formel:

$$A_{(\lambda)th} = w * 1/100 * \sum_{n=1}^{n=p} K_{\lambda(n)} * a_{(n)} \qquad [1]$$

wobei n für die Anzahl der UV-Filtersubstanzen von n=1 bis n=p, w für die anfängliche Oberflächenkonzentration der Sonnenschutzzubereitung unmittelbar nach Auftragung auf einen rauhen, transparenten Träger in $mg/cm^2$, $K_{\lambda(n)}$ für das Absorptionsvermögen der Anzahl n der UV-Filtersubstanzen bei einer Wellenlänge $\lambda$ und $a_{(n)}$ für den Anteil der UV-Filtersubstanzen an der Sonnenschutzzubereitung in Gewichtsprozenten steht;

(3) Bestimmung der experimentellen UV-Absorptionen $A_{(\lambda)exp}$ von 290 bis 400 nm, in Wellenlängenschritten im Bereich von 1-10 nm, der UV-Filtersubstanzen oder der Gemische der Substanzen aus Schritt (2), die in demselben Anteil und in derselben Basisformulierung, wie in Schritt (2) vorliegen, wobei die Basisformulierung aus einer Gruppe vom Typ Öl/Wasser-Emulsionen, Wasser/Öl-Emulsionen, Öl, Gel, Stiftmasse, Aerosol und Salbe ausgewählt ist und wobei die Mischung auf einem rauhen, transparenten Träger aufgetragen ist, um einen ungleichmäßigen Film mit einer Oberflächendichte von w $mg/cm^2$ zu erhalten;

(4) Auftragung von Wertepaaren der experimentellen Absorptionsdaten $A_{(\lambda)exp}$ aus Schritt (3) und der theoretischen Absorptionsdaten $A_{(\lambda)th}$ aus Schritt (2) bei derselben Wellenlänge in einen Graphen;

(5) Bestimmung einer mathematischen Funktion, die in Korrelation mit dem Graphen aus Schritt (4) steht, **durch** Aufnahme von Parametern über einen rauhen Film, wobei der Film ein mathematisches Modell für den Einsatz einer Sonnenschutzzubereitung auf einem transparenten Träger, wie aus Schritt (3), beschreibt und wobei **durch** Optimierung des Näherungsansatzes an die Wertepaare der Absorptionsdaten, $A_{(\lambda)exp}$ gegen $A_{(\lambda)th}$, mittels einer Näherungsmethode verschiedene Teilareale $f_{(1)}$ bis $f_{(i)}$ mit verschiedenen Oberflächedichten $w_{(1)}$ bis $w_{(i)}$ berechnet werden, mit dem Ansatz:

$$\sum_{n=1}^{n=i} f(n) = 1$$

und

$$\sum_{n=1}^{n=i} f(n) * w(n) = w$$

wobei w für die anfängliche Oberflächendichte des Sonnenschutzmittels unmittelbar nach der Auftragung auf den transparenten Träger steht, und wobei für den Näherungsansatz gilt:

$$A_{(\lambda)exp} = -\log\left[\sum_{n=1}^{n=i} f(n) * 10^{-w(n) * A(\lambda)th/w}\right] \quad [2],$$

wobei $A_{(\lambda)th}$ für die berechnete theoretische Absorption nach Schritt (2) und $A_{(\lambda)exp}$ für die experimentelle Absorption nach Schritt (3) steht;

(6) Verwendung des in den Schritten (1) bis (5) beschriebenen Verfahrens zur Voraussage realistischer Sonnenschutzfaktoren von Sonnenschutzmitteln, vor der experimentellen in vitro oder in vivo Bestimmung, die die folgenden Schritte umfasst:

(7) Berechnung der theoretischen UV-Absorption $A_{(\lambda)th}$ von 290 bis 400 nm, in Wellenlängenschritten im Bereich von 1-10 nm, einer UV-Filtersubstanz oder eines Gemisches von Substanzen, die in Gewichtsprozentanteilen $a_1$ bis $a_n$ in dem Sonnenschutzmittel nach Schritt (6) enthalten sind, nach Formel [1] aus Schritt (2);

(8) Berechnung einer realistischen UV-Absorption $A_{(\lambda)real}$ von 290 bis 400 nm, in Wellenlängenschritten im Bereich von 1-10 nm, entsprechend der theoretischen UV-Absorption aus Schritt (7) und dem Näherungsansatz [2] aus Schritt (5), in dem die Teilareale $f_{(1)}$ bis $f_{(i)}$ und die Oberflächedichten $w_{(1)}$ bis $w_{(i)}$ zuvor nach Schritt (5) für eine Basisformulierung gleichen Typs, wie bei der zu untersuchenden Sonnenschutzzubereitung, bestimmt werden mit der Gleichung

$$A_{(\lambda)real} = -\log\left[\sum_{n=1}^{n=i} f(n) * 10^{-w(n) * A(\lambda)th/w}\right] \quad [2a];$$

(9) Berechnung von realistischen Sonnenschutzfaktoren (SPF) oder Breitbandspektrenindizes auf Basis der im Schritt (8) ermittelten realistischen UV-Absorption $A_{(\lambda)real}$ mit bekannten Gleichungsansätzen.

2. Verfahren nach Anspruch 1, **dadurch gekennzeichnet, daß** die Berechnung der realistischen Sonnenschuthkoren der Sonnenschutzzubereitung aus Schritt (9) nach der Gleichung:

$$SPF = \frac{\sum_{290}^{400} E_\lambda * I_\lambda * \Delta\lambda}{\sum_{290}^{400} E_\lambda * I_\lambda * T_\lambda * \Delta\lambda} \quad [3]$$

erfolgt, wobei $T_\lambda$, erhältlich über den Zusammenhang $T_\lambda = 10^{-A(\lambda)real}$, die Sonnenschutzdurchlässigkeit bei der Wellenlänge $\lambda$ darstellt, $E_\lambda$ ist die Strahhmgsdichte terrestrischen Sonnenlichts der Wellenlänge $\lambda$, bei klarem Himmel, Mittags am Mittsommer und bei einer geographischen Breite von 40° N und $I_\lambda$ ist das aktive Erythemspektrum.

3. Verfahren nach Anspruch 1, **dadurch gekennzeichnet, daß** die Wellenlängenschritte 5 nm betragen.

4. Verfahren nach Anspruch 1, **dadurch gekennzeichnet, daß** die Näherungsmethode in Schritt (5) die Näherungsmethode der kleinsten Quadrate ist.

5. Verfahren nach Anspruch 1, **dadurch gekennzeichnet, daß** i in Gleichung [2] den Wert 3, mit 3 berechneten Teilarealen $f_{(1)}$, $f_{(2)}$ und $f_{(3)}$ und 3 berechneten Oberflächendichten $w_{(1)}$, $w_{(2)}$ und $w_{(3)}$, annimmt.

6. Verfahren nach Anspruch 1, **dadurch gekennzeichnet, daß** der transparente Träger eine ausgeschnittene, menschliche Epidermis ist oder aus einer Gruppe ausgewählt wird, die künstlichen Hautersatz umfasst, wie chirurgisches Band, hydrierten Kollagenfilm, aufgerauhte Quarzplatten oder aufgerauhte Polymethylmethacrylat-Platten (PMMA) umfaßt.

7. Verfahren nach Anspruch 1, **dadurch gekennzeichnet, daß** in Schritt (5) die verschiedenen Teilareale ($f_1$) to ($f_i$) ersetzt weden durch eine unbestimmte Zahl von Teilarealen, deren Summe F ist, der kumulative Anteil des Teilareals, der eine Zahl zwischen 0 und 1 ist, wobei gilt:

$$\int_0^1 dF = 1 \quad \text{und} \quad \int_0^1 W_{(F)} \times dF = w \quad ,$$

worin w die anfängliche Oberflächendichte des Sonnenschutzmittels ist nach Auftragung auf den transparenten Träger, $W_{(F)}$ ist die Oberflächendichtefunktion, and wobei der Näherungsansatz gilt:

$$A_{(\lambda)exp} = -\log [\int_0^1 10^{-W_{(F)} \times A_{\lambda th}/w} \times dF] \qquad [2b]$$

worin $A_{\lambda\ th}$ und $A_{(\lambda)exp}$ die oben genannte Bedeutung haben.

8. Verfahren nach Anspruch 1, **dadurch gekennzeichnet, daß** die Berechnung der realistischen UVA-Schutzfaktoren (PF UVA) einer Sonnenschutzzubereitung aus Schritt (9) nach der Gleichung

$$\text{PF UVA} = \frac{\sum_{320}^{400} E_\lambda * I_\lambda * \Delta\lambda}{\sum_{320}^{400} E_\lambda * I_\lambda * T_\lambda * \Delta\lambda} \qquad [4]$$

erfolgt mit denselben Definitionen wie in Anspruch 2, außer für $I_\lambda$, das für das biologische UVA-Anregungsspektrum steht.

9. Verfahren nach Anspruch 1, **dadurch gekennzeichnet, dass** die Berechnung realistischer UVA /UVB-Verhältnisse der Sonnenschutzzubereitung aus Schritt (9) nach der Gleichung

$$\text{UVA / UVB} = \frac{\sum_{320}^{400} A_{\lambda\,real} * \Delta\lambda}{\sum_{290}^{320} A_{\lambda\,real} * \Delta\lambda} \qquad [5]$$

erfolgt mit denselben Definitionen wie zuvor beschrieben.

10. Verfahren nach Anspruch 1, **dadurch gekennzeichnet, dass** die UV-Filtersubsubstanzen aus der Gruppe ausgewählt werden, die Octyl Methoxycinnamate, Octyocrylene, Octylsalicylate, Penylbenzylimidazole Sulfonic Acid, 4-Methylbenzylidene Camphor, Dioctyl Butamido Triazone, Isoamyl-p-methoxycinnamat, Benzophenone-3, Benzophenone-4, Butyl-Methoxydibenzoylmethane, Methylene Bis-Benzoyltriazoyl Tetramethylbutylphenol, Homosalate, Para Amino benzoic Acid (PABA), Octyl Dimethyl PABA, Menthyl Anthranilate (alles INCI Namen) und Gemische derselben umfassen.

## Claims

1. Method for determination of realistic UV protection factors or bread spectrum indices of a sunscreen preparation, which comprises:

   (1) Determination of absorptivities of pure known UV filter substances in a pure solvent or a mixture thereof, at wavelengths from 290 to 400 nm, in wavelength steps in the range of 1-10 nm;

   (2) Calculation of the theoretical UV absorbances $A_{(\lambda)th}$, from 290 to 400 nm, in wavelength steps in the range of 1-10 nm , of some pure UV filter substances or mixtures thereof which are contained in different amounts in a defined basic formulation, according to application of Beer-Lambert law to the absorptivities previously determined in step (1) by using the following equation:

$$A_{(\lambda)th} = w * 1/100 * \sum_{n=1}^{n=p} K_{\lambda(n)} * a_{(n)} \quad [1]$$

   wherein
   n is the number of UV filter substances from n=1 to n= p, w is the initial surface density of the sunscreen preparation immediately after deposition on an uneven transparent substrate in $mg/cm^2$, $K_{\lambda(n)}$ is the absorptivity of the number n of UV filter substances at a wavelength $\lambda$ and a(n) is the amount of the UV filter substance in the sunscreen preparation in % by weight;

   (3) Determination of the experimental UV absorbances $A_{(\lambda)exp}$, from 290 to 400 nm, in wavelength steps in the range of 1-10 nm, of the UV filter substance or mixtures of substances of step (2), which has the same amount and is in the same basic formulation such as in step (2), which formulation is selected from the group consisting of the type O/W, W/O, oil, gel, stick, mousse, aerosol and ointment and wherein the formulation is deposited on an uneven transparent substrate, to achieve an irregular film of a surface density of w $mg/cm^2$;

   (4) Applying of pairs of experimental absorbance data $A_{(\lambda)exp}$ of step (3) and theoretical absorbance data $A_{(\lambda)th}$ of step (2) of the same wavelength in a graph;

   (5) Determination of a mathematical function in correlation with the graph of step (4) by incorporation of parameters of an uneven film, which film represents a mathematical model of the application of a sunscreen preparation on a transparent substrate such as in step (3) and wherein by optimizing the fit to the pairs of absorbance data, $A_{(\lambda)exp}$ versus $A_{(\lambda)th}$, through an error assessment, different area fractions $f_{(1)}$ to $f_{(i)}$ with different surface densities $w_{(1)}$ to w(i) are calculated, whereby applies:

$$\sum_{n=1}^{n=i} f(n) = 1$$

   and

$$\sum_{n=1}^{n=i} f(n).w(n) = w$$

   wherein w is the initial surface density of the sunscreen preparation immediately after deposition on the transparent substrate, and wherein the mathematical function is:

$$A_{(\lambda)exp} = -\log \left[ \sum_{n=1}^{n=i} f(n).10^{-w(n).A(\lambda)th/w} \right] \qquad [2],$$

wherein $A_{(\lambda)th}$ is the calculated theoretical absorbance according to step (2) and $A_{(\lambda)exp}$ is the experimental absorbance according to step (3);

(6) Application of the method described in steps 1 to 5 to the prediction of realistic sun protection factors of a sunscreen preparation, previously to achieve any experimental in vitro or in vivo determination, wherein the following steps are involved:

(7) Calculation of the theoretical UV absorbances $A_{(\lambda)th}$, from 290 to 400nm, in wavelength steps in the range of 1-10 nm, of an UV filter substance or mixture of substances which are contained in wt% amounts $a_1$ to $a_n$ in the sunscreen preparation of step (6), according to equation [1] of step (2);

(8) Calculation of realistic UV absorbances $A_{(\lambda)real}$, from 290 to 400 nm, in wavelength steps in the range of 1-10 nm, according to the theoretical UV absorbances calculated in step (7) and the mathematical function [2] of step (5),in which the area fractions $f_{(1)}$ to $f_{(i)}$ and the surface densities $w_{(1)}$ to $w_{(i)}$ were previously determined in step (5) for a basic formulation of the same type as the sunscreen preparation under study, by equation

$$A_{(\lambda)real} = -\log \left[ \sum_{n=1}^{n=i} f(n).10^{-w(n).A(\lambda)th/w} \right] \qquad [2a]$$

(9) Calculation of realistic sun protection factors(SPF) or bread spectrum indices on basis of the realistic UV absorbance $A_{(\lambda)real}$ determined in step (8), with known equations.

**2.** Method according to claim 1, wherein the calculation of realistic sun protection factors of the sunscreen preparation of step (9) follows according to the equation:

$$SPF = \frac{\sum_{290}^{400} E_\lambda . I_\lambda . \Delta\lambda}{\sum_{290}^{400} E_\lambda . I_\lambda . T_\lambda . \Delta\lambda} \qquad [3]$$

wherein $T\lambda$ is the sunscreen transmittance at wavelength $\lambda$, whereby applies $T_\lambda = 10^{-A(\lambda)real}$, $E_\lambda$ is the spectral irradiance of terrestrial sunlight at wavelength $\lambda$ expected for a clear sky at noon in midsummer for a latitude of 40° N, $I_\lambda$ is the erythema action spectrum.

**3.** Method according to claim 1, wherein the wave length steps are 5 nm.

**4.** Method according to claim 1, wherein the error assessment in step (5) is the least squares error assessment.

**5.** Method according to claim 1, wherein i in equation [2] has a value of 3, with 3 calculated area fractions, $f_{(1)}$, $f_{(2)}$ and $f_{(3)}$ and 3 calculated surface densities $w_{(1)}$ $w_{(2)}$ and $w_{(3)}$.

**6.** Method according to claim 1, wherein the transparent substrate is excised human epidermis or is selected from the group consisting of synthetic skin substitutes like surgical tape, hydrated collagen film, roughened quartz plate or roughened polymethylmethacrylate plate (PMMA).

**7.** Method according to claim 1, wherein in step (5) the different area fractions ($f_1$) to ($f_i$) are replaced by an infinite

number F, cumulative fraction of unit area, which is a number between 0 and 1, whereby applies:

$$\int_0^1 dF = 1 \quad \text{and} \quad \int_0^1 W_{(F)} \times dF = \mathrm{w} \quad ,$$

wherein w is the initial surface density of the sunscreen preparation deposited on the transparent substrate, $W_{(F)}$ being the surface density function, and wherein the mathematical function becomes:

$$A_{(\lambda)\exp} = -\log \left[ \int_0^1 10^{-W_{(F)} \times A\lambda th / w} \times dF \right] \qquad [2b]$$

and wherein $A_{\lambda\ th}$ and $A_{(\lambda)\exp}$ have the same meaning as above.

**8.** Method according to claim 1, wherein the calculation of realistic UVA protection factors (PF UVA) of the sunscreen preparation of step (9) is made according to the equation

$$\text{PF UVA} = \frac{\sum_{320}^{400} E_\lambda . I_\lambda . \Delta\lambda}{\sum_{320}^{400} E_\lambda . I_\lambda . T_\lambda . \Delta\lambda} \qquad [4]$$

with the same definitions as previously in step (9), excepted for $I_\lambda$, which is a biological UVA action spectrum.

**9.** Method according to claim 1, wherein the calculation of realistic UVA / UVB ratio of the sunscreen preparation of step (9) is made according to the equation:

$$\text{UVA / UVB} = \frac{\sum_{320}^{400} A_{\lambda\ real} . \Delta\lambda}{\sum_{290}^{320} A_{\lambda\ real} . \Delta\lambda} \qquad [5]$$

with the same definitions as previously mentioned.

**10.** Method according to claim 1, wherein the organic UV filter substances are selected from the group consisting of Octyl Methoxycinnamate, Octocrylene, Octyl Salicylate, Phenylbenzylimidazole Sulphonic Acid, 4-Methylbenzylidene Camphor; Dioctyl Butamido Triazone; Isoamyl p-Methoxycinnamate, Benzophenone-3, Benzophenone-4, Butyl-Methoxydibenzoylmethane, Methylene Bis-Benzoyltriazoyl Tetramethylbutylphenol, Homosalate, Para Amino Benzoic Acid (PABA), Octyl Dimethyl PABA, Menthyl Anthranilate (all INCI names) and mixtures thereof.

**Revendications**

**1.** Procédé de détermination d'indices de protection UV réalistes ou d'indices de spectre à large bande d'une préparation de protection solaire, **caractérisé par** les étapes suivantes :

(1) détermination du pouvoir d'absorption de substances filtrant les UV organiques ou inorganiques connues

pures dans un solvant organique pur ou un mélange le contenant, à une longueur d'onde de 290 à 400 nm, par incréments de longueur d'onde dans la plage de 1 à 10 nm ;
(2) calcul de l'absorption UV théorique $A_{(\lambda)th}$ de 290 à 400 nm, par incréments de longueur d'onde de 5 nm, de quelques substances filtrant les UV pures, ou de leurs mélanges, contenues à des teneurs différentes dans une formulation de base définie, correspondant à l'application de la loi de Lambert-Beer au pouvoir d'absorption déterminé à l'étape (1) précédente en utilisant la formule suivante :

$$A_{(\lambda)th} = w * 1/100 * \sum_{n=1}^{n=p} K_{\lambda(n)} * a_{(n)} \qquad [1]$$

où n est le nombre des substances filtrant les UV de n=1 à n=p, w est la concentration superficielle initiale de la préparation de protection solaire immédiatement après son application sur un support transparent rugueux en mg/cm$^2$, $K_{\lambda(n)}$ est le pouvoir d'absorption du nombre n des substances filtrant les UV à une longueur d'onde $\lambda$ et $a_{(n)}$ est la teneur en substances filtrant les UV dans la préparation de protection solaire en pourcentage en poids ;
(3) détermination des absorptions UV expérimentales $A_{(\lambda)exp}$ de 290 à 400 nm, par incréments de longueur d'onde dans la plage de 1 à 10 nm, des substances filtrant les UV ou des mélanges de substances de l'étape (2) qui sont présents avec la même teneur et dans la même formulation de base qu'à l'étape (2), la formulation de base étant choisie dans un groupe comprenant des émulsions d'huile dans l'eau, des émulsions d'eau dans d'huile, une huile, un gel, une pâte pour sticks, une mousse, un aérosol et une crème, et le mélange étant appliqué sur un support transparent rugueux afin d'obtenir un film ayant une densité superficielle de w mg/cm$^2$ ;
(4) report d'une paire de valeurs des données d'absorption expérimentales $A_{(\lambda)exp}$ de l'étape (3) et des données d'absorption théoriques $A_{(\lambda)th}$ de l'étape (2) à la même longueur d'onde sur un graphique ;
(5) détermination d'une fonction mathématique corrélée avec le graphique de l'étape (4) en relevant des paramètres sur un film rugueux, le film décrivant un modèle mathématique pour l'utilisation d'une préparation de protection solaire sur un support transparent, comme à l'étape (3), et différents domaines partiels $f_{(1)}$ à $f_{(i)}$ ayant différentes densités superficielles $w_{(1)}$ à $w_{(i)}$ étant calculés en optimisant l'équation d'ajustement des paires de valeurs des données d'absorption, $A_{(\lambda)exp}$ contre $A_{(\lambda)th}$, par la méthode d'ajustement des moindres carrés avec l'équation :

$$\sum_{n=1}^{n=i} f(n) = 1,$$

et

$$\sum_{n=1}^{n=i} f(n) * w(n) = w$$

où w est la densité superficielle initiale du produit de protection solaire immédiatement après son application sur le support transparent, l'équation d'ajustement s'écrivant :

$$A_{(\lambda)exp} = -\log \left[\sum_{n=1}^{n=i} f(n) * 10^{-w(n) * A_{(\lambda)th}/w}\right] \qquad [2]$$

où $A_{(\lambda)th}$ est l'absorption théorique calculée suivant l'étape (2) et $A_{(\lambda)exp}$ est l'absorption expérimentale suivant l'étape (3) ;
(6) utilisation du procédé décrit dans les étapes (1) à (5) pour prédire des indices de protection solaire réalistes

pour des préparations de protection solaire, avant la détermination expérimentale *in vitro* ou *in vivo,* qui comprend les étapes suivantes :

(7) calcul de l'absorption UV théorique $A_{(\lambda)th}$ de 290 à 400 nm, par incréments de longueur d'onde dans la plage de 1 à 10 nm, d'une substance filtrant les UV ou d'un mélange de substances contenues avec des teneurs en pourcentage en poids $a_1$ à $a_n$ dans la préparation de protection solaire de l'étape (6), suivant la formule [1] de l'étape (2) ;

(8) calcul d'une absorption UV réaliste $A_{(\lambda)réal}$ de 290 à 400 nm, par incréments de longueur d'onde dans la plage de 1 à 10 nm, correspondant aux absorptions UV théoriques de l'étape (7) et à l'équation d'ajustement [2] de l'étape (5), dans laquelle les domaines partiels $f_{(1)}$ à $f_{(i)}$ et les densités superficielles $w_{(1)}$ à $w_{(i)}$ sont calculés auparavant suivant l'étape (5) pour une formulation de base du même type, comme pour la préparation de protection solaire à analyser :

$$A_{(\lambda)réal} = -\log \left[ \sum_{n=1}^{n=i} f(n) * 10^{-w(n)*A(_{\lambda})th/w} \right] \quad [2a]$$

(9) calcul d'indices de protection solaire (IPS) réalistes ou d'indices de spectre à large bande sur la base de l'absorption réaliste $A_{(\lambda)réal}$ déterminée à l'étape (8) au moyen de jeux d'équations connus.

**2.** Procédé selon la revendication 1, **caractérisé en ce que** le calcul des indices de protection solaire réalistes de la préparation de protection solaire de l'étape (9) se fait au moyen de l'équation :

$$IPS = \frac{\sum_{290}^{400} E_\lambda * I_\lambda * \Delta\lambda}{\sum_{290}^{400} E_\lambda * I_\lambda * T_\lambda * \Delta\lambda} \quad [3]$$

où $T_\lambda$, qui peut être tiré de la relation $T_\lambda = 10^{-A(\lambda)réal}$, est la perméabilité du produit de protection solaire à la longueur d'onde $\lambda$, $E_\lambda$ est la densité du rayonnement de la lumière solaire terrestre de longueur d'onde $\lambda$ par temps clair le midi en plein été et à une latitude géographique de 40° N, et $I_\lambda$ est le spectre d'érythème actif.

**3.** Procédé selon la revendication 1, **caractérisé en ce que** les incréments de longueur d'onde sont de 5 nm.

**4.** Procédé selon la revendication 1, **caractérisé en ce que** la méthode d'ajustement de l'étape (5) est la méthode d'ajustement des moindres carrés.

**5.** Procédé selon la revendication 1, **caractérisé en ce que** i dans l'équation [2] prend la valeur 3 pour 3 domaines partiels calculés $f_{(1)}$, $f_{(2)}$ et $f_{(3)}$ et 3 densités superficielles calculées $W_{(1)}$, $W_{(2)}$ et $W_{(3)}$.

**6.** Procédé selon la revendication 1, **caractérisé en ce que** le support transparent est un morceau d'épiderme humain ou bien est choisi dans un groupe comprenant des substituts artificiels de la peau tels qu'une bande chirurgicale, un film de collagène hydraté, des plaques de quartz dépoli ou des plaques de polyméthacrylate de méthyle (PMMA) dépoli.

**7.** Procédé selon la revendication 6, **caractérisé en ce que** le support transparent est une bande chirurgicale Transpore® qui permet d'obtenir des valeurs d'IPS *in vitro* proches des valeurs d'IPS *in vivo.*

**8.** Procédé selon la revendication 1, **caractérisé en ce que** le calcul des indices de protection UVA (IP UVA) réalistes d'une préparation de protection solaire de l'étape (9) se fait au moyen de l'équation

$$IP\ UVA = \frac{\sum_{320}^{400} E_\lambda * I_\lambda * \Delta\lambda}{\sum_{320}^{400} E_\lambda * I_\lambda * T_\lambda * \Delta\lambda} \qquad [4]$$

avec les mêmes définitions que dans la revendication 2, sauf pour $I_\lambda$, qui représente le spectre d'excitation UVA biologique.

**9.** Procédé selon la revendication 1, **caractérisé en ce que** le calcul de ratios UVA/UVB réalistes pour la préparation de protection solaire de l'étape (9) se fait au moyen de l'équation

$$UVA/UVB = \frac{\sum_{320}^{400} A_{\lambda réal} * \Delta\lambda}{\sum_{290}^{320} A_{\lambda réal} * \Delta\lambda} \qquad [5]$$

avec les mêmes définitions que celles décrites précédemment.

**10.** Procédé selon la revendication 1, **caractérisé en ce que** les substances filtrant les UV sont choisies dans le groupe comprenant le méthoxycinnamate d'octyle, l'octyocrylène, le salicylate d'octyle, l'acide phénylbenzylimidazole sulfonique, le 4-méthylbenzylidènecamphre, la dioctylbutamidotriazone, le p-méthoxycinnamate d'isoamyle, la benzophénone-3, la benzophénone-4, le butylméthoxydibenzoylméthane, le méthylène-bis-benzoyltriazoyltétraméthylbutylphénol, l'homosalate, l'acide paraaminobenzoïque (PABA), le para-aminobenzoate d'octyldiméthyle, l'anthranilate de menthyle (tous des désignations INCI) et des mélanges de ceux-ci.

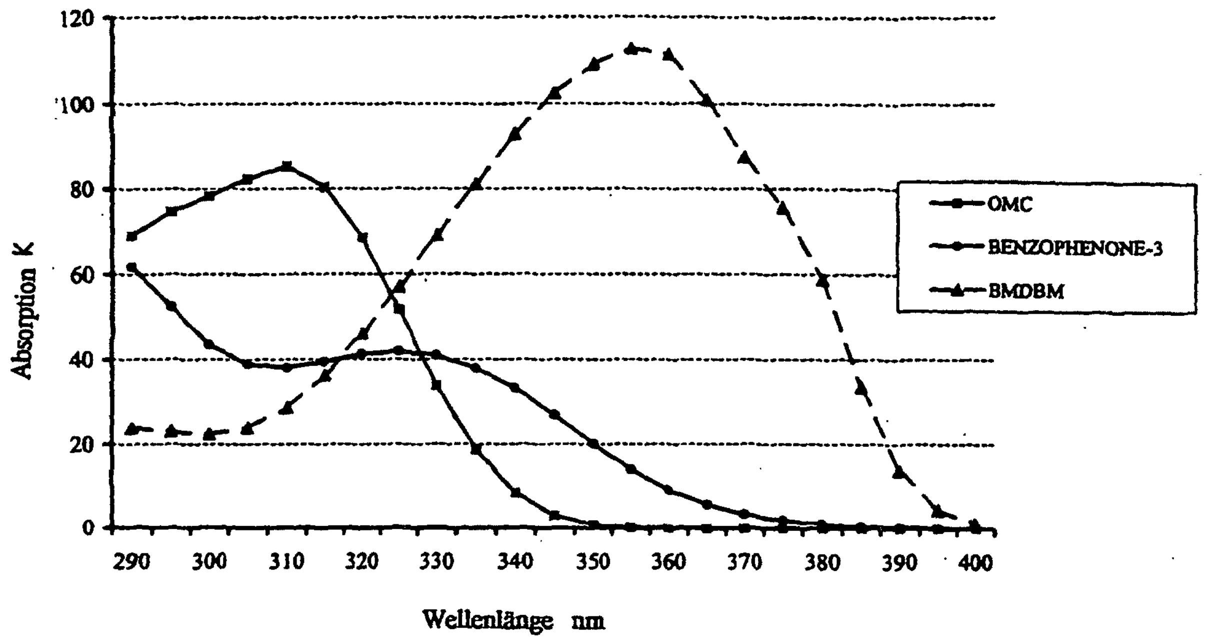
120
100
80
60
40
20
0
Absorption K
290
300
310
320
330
340
350
360
370
380
390
400
Wellenlänge nm
OMC
BENZOPHENONE-3
BMDBM

Fig. 1

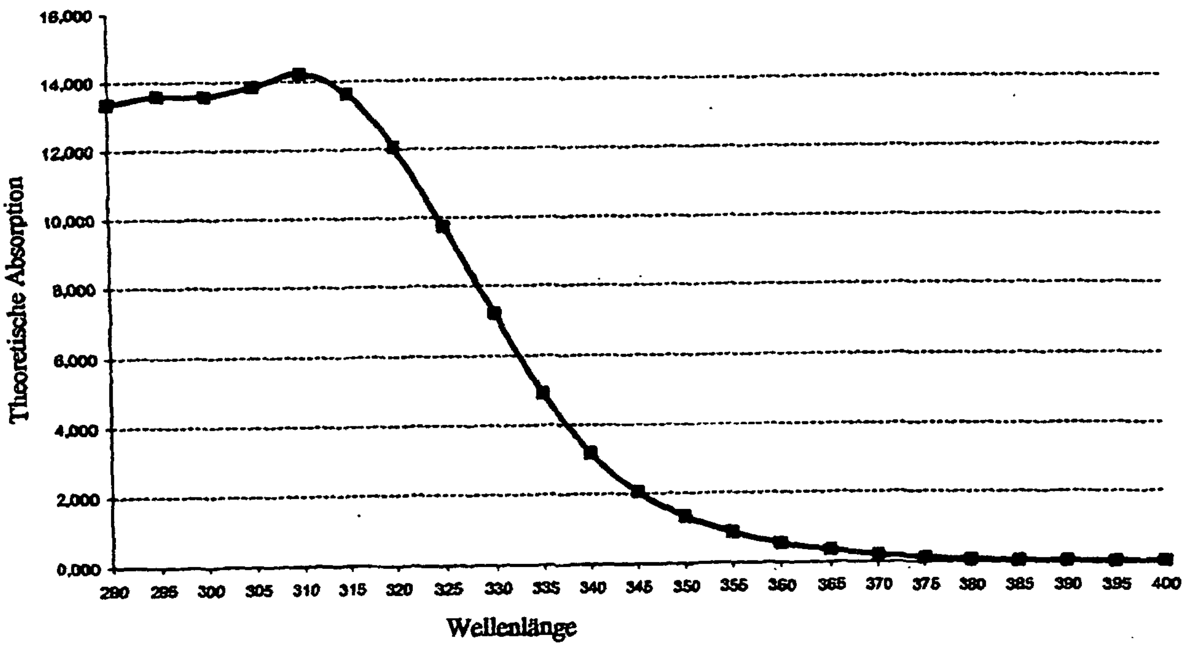
16,000
14,000
12,000
10,000
8,000
6,000
4,000
2,000
0,000
Theoretische Absorption
290
295
300
305
310
315
320
325
330
335
340
345
350
355
360
365
370
375
380
385
390
395
400
Wellenlänge

Fig. 2

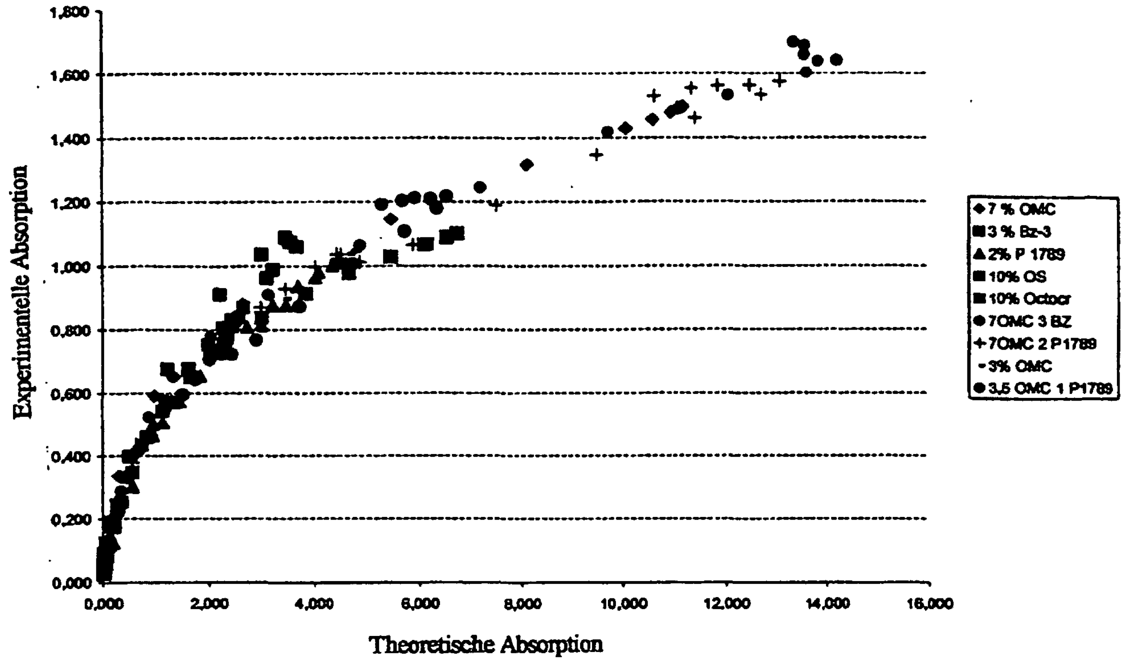
1,800
1,600
1,400
1,200
1,000
0,800
0,600
0,400
0,200
0,000
Experimentelle Absorption
0,000
2,000
4,000
6,000
8,000
10,000
12,000
14,000
16,000
Theoretische Absorption
7 % OMC
3 % Bz-3
2% P 1789
10% OS
10% Octocr
7OMC 3 BZ
7OMC 2 P1789
3% OMC
3,5 OMC 1 P1789

Fig. 3

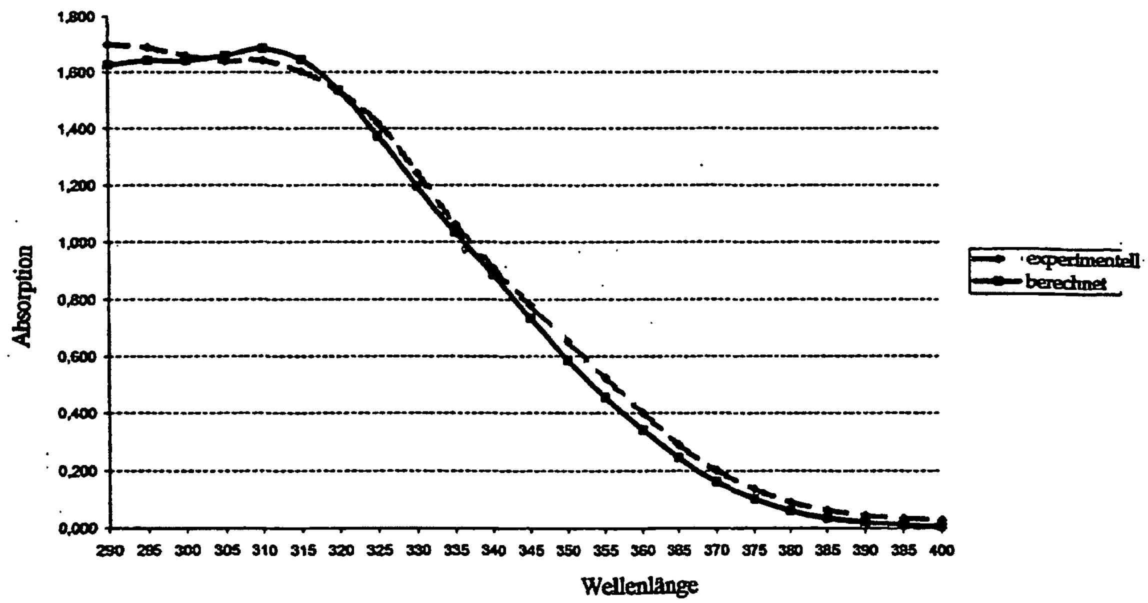
1,800
1,600
1,400
1,200
1,000
0,800
0,600
0,400
0,200
0,000
Absorption
290 295 300 305 310 315 320 325 330 335 340 345 350 355 360 365 370 375 380 385 390 395 400
Wellenlänge
experimentell
berechnet

Fig. 4